# EUROPÄISCHE PATENTSCHRIFT

(11) **EP 2 188 614 B1**
(45) Veröffentlichungstag und Bekanntmachung des Hinweises auf die Patenterteilung: **17.08.2011**
(21) Anmeldenummer: 08801754.6
(22) Anmeldetag: 29.08.2008
(51) Int. Cl.: G01N 21/19

(54) **VERFAHREN UND VORRICHTUNG ZUR DETEKTION VON VERUNREINIGUNGEN IN LÄNGSBEWEGTEM GARN**
METHOD AND DEVICE FOR DETECTING IMPURITIES IN LONGITUDINALLY MOVING YARN
PROCÉDÉ ET DISPOSITIF DE DÉTECTION D'IMPURETÉS DANS DU FIL DÉPLACÉ LONGITUDINALEMENT

(30) Priorität: 12.09.2007 DE 102007043353
(43) Veröffentlichungstag der Anmeldung: 26.05.2010
(73) Patentinhaber: Oerlikon Textile GmbH & Co. KG, 42897 Remscheid (DE)
(72) Erfinder: BEGENDZOV, Waldemar, 41239 Mönchengladbach (DE); BIRLEM, Olav, 41812 Erkelenz (DE); BOLTEN, Daniela, 41812 Erkelenz (DE); FECHTER, Ulrich, 41236 Mönchengladbach (DE); FINGEL, Oliver, 47608 Geldern (DE); HENKE, Marc, 30627 Hannover (DE); HÜLS, Jürgen, 40670 Meerbusch (DE); KRÜGER, Andreas, 41065 Mönchengladbach (DE); MOHR, Hans-Peter, 41189 Mönchengladbach (DE); RADERMACHER, Wolfgang, 41844 Wegberg (DE)
(74) Vertreter: Hamann, Arndt
(86) Internationale Anmeldenummer: PCT/EP2008/007074
(87) Internationale Veröffentlichungsnummer: WO 2009/036873

(56) Entgegenhaltungen:
- EP-A- 0 553 446
- WO-A-93/13407
- DE-A1- 10 009 131
- US-A- 5 414 520

## Beschreibung

Die Erfindung betrifft ein Verfahren zur Detektion von Verunreinigungen, insbesondere von Fremdfasern, in längsbewegtem Garn, wobei Licht in Richtung des Garns emittiert wird und wobei ein Remissionswert, der die Intensität des vom Garn remittierten Lichtes repräsentiert, und ein zugehöriger Transmissionswert, der die Intensität des vom Garn transmittierten Lichtes repräsentiert, ermittelt wird. Ferner betrifft die Erfindung eine Vorrichtung zur Detektion von Verunreinigungen, insbesondere von Fremdfasern, in längsbewegtem Garn mit einer Steuer- und Auswerteeinrichtung und mindestens einer Lichtquelle, die dazu ausgebildet ist, Licht in Richtung des Garns zu emittieren, sowie Sensoren zur Erfassung eines Remissionswertes, der die Intensität des vom Garn remittierten Lichtes repräsentiert, und eines zugehörigern Transmissionswertes, der die Intensität des vom Garn transmittierten Lichtes repräsentiert.

Vor der Herstellung von Fäden und Garnen aus Baumwolle oder anderen Naturfasern findet zunächst eine mechanische Reinigung der Fasern statt. Dadurch werden grobe Verunreinigungen aus den Fasern entfernt. Anderseits gibt es gewisse Verunreinigungen, wie zum Beispiel Fremdfasern verschiedener Art, die sich auf diese Weise nicht entfernen lassen. Ferner können die Verunreinigungen beziehungsweise Fremdfasern erst im weiteren Verarbeitungsprozess auftreten. Diese werden dann in den Faden oder das Garn eingesponnen und beinträchtigen die nachfolgenden Bearbeitungsprozesse sowie die Qualität des Endproduktes. Genauso wie bei Naturfasern können auch bei Chemiefasern nicht wünschenswerte Verunreinigungen im Garn auftreten.

Bekannte optische Verfahren zur Detektion von Verunreinigungen in längsbewegtem Garn beruhen dabei auf der Auswertung des vom Garn reflektierten beziehungsweise remittierten Lichtes. Fremdfasern verändern das Remissionsverhalten des Garns. Je nach Art der Fremdfasern werden mehr oder weniger Lichtanteile beziehungsweise Wellenlängebereiche des Lichtes absorbiert, so dass umgekehrt weniger oder mehr Lichtanteile reflektiert werden. Neben dem Anteil des absorbierten Lichtes hat allerdings noch der Durchmesser des Garns Einfluss auf die Intensität des reflektierten Lichtes, so dass eine Dick- oder Dünnstelle auch die Intensität des reflektierten Lichtes beeinflusst und damit das Signal zur Erkennung von Fremdfasern verfälscht. Der Durchmesser des Garns bestimmt ferner die Intensität des transmittierten Lichtes.

In der EP 0 553 446 B1 ist eine Möglichkeit offenbart, den Durchmessereinfluss des Reflektionssignals zu eliminieren. Dazu wird das Reflexionssignal, dessen Größe vom Auftreten einer Verunreinigung abhängig ist, direkt mathematisch mit dem Transmissionssignal, das nur vom Durchmesser des Garns beeinflusst wird, verknüpft. Als mathematische Verknüpfung ist dabei die Bildung eines Quotienten aus Reflektions- und Transmissionssignal offenbart, um ein durchmesserunabhängiges Fremdfaser-Messsignal zu erhalten. Das Messsignal stellt den Helligkeitswert des Garns dar.

Ein solches Verfahren geht davon aus beziehungsweise funktioniert nur dann zufrieden stellend, wenn das Reflektionsverhalten über den gesamten Durchmesser des Garns identisch ist und damit die Intensität des vom nicht verunreinigten Garn reflektierten Lichtes direkt proportional zum Durchmesser des Garns ist. Dies ist jedoch nur näherungsweise erfüllt. Das vom Garn reflektierte Licht hängt noch von einer Vielzahl anderer Faktoren ab, so spielt zum Beispiel die Garnstruktur und die Homogenität des in Richtung des Garns emittierten Lichtes eine Rolle. Auch ist es nachvollziehbar, dass an dem Garn das Reflektionsverhalten am Rand anders ist, als in der Mitte.

Es ist daher die Aufgabe der vorliegenden Erfindung, die Detektion von Verunreinigungen in längsbewegtem Garn zu verbessern und die Auswertung der Remissions- und Transmissionssignale so zu gestalten, dass sonstige Einflüsse weitestgehend kompensiert werden.

Die Aufgabe wird erfindungsgemäß durch die kennzeichnenden Merkmale des Verfahrensanspruches 1 sowie des Vorrichtungsanspruches 17 gelöst. Vorteilhafte Weiterbildungen der Erfindung sind Gegenstand der Unteransprüche.

Zur Lösung der Aufgabe wird vorgeschlagen, dass dem jeweiligen Transmissionswert ein remissionsabhängiger Referenzwert zugeordnet wird und der aktuelle Remissionswert mit dem Referenzwert verglichen wird oder dass dem jeweiligen Remissionswert ein transmissionsabhängiger Referenzwert zugeordnet wird und der aktuelle Transmissionswert mit dem Referenzwert verglichen wird.

Durch die Zuordnung eines Referenzwertes für die Remission beziehungsweise für die Transmission zu jedem auftretenden Transmissionswert beziehungsweise Remissionswert wird die bekannte Durchmesserabhängigkeit der Messsignale nicht nur abgeschwächt, sondern weitestgehend eliminiert. Der Referenzwert ist an die jeweiligen Bedingungen und das jeweilige Garn angepasst, so dass auch sonstige Einflussfaktoren kompensiert werden können. Im Unterschied zum Stand der Technik wird also die Durchmesserabhängigkeit des Remissionssignals nicht durch die Bestimmung eines Helligkeitswertes erreicht, sondern durch Vergleich mit dem Referenzwert.

In einer Weiterbildung der Erfindung ist der Referenzwert ein Mittelwert von Remissionswerten beziehungsweise von Transmissionswerten. Vorteilhafterweise werden alle während einer Referenzlänge des Garns zu dem gleichen Transmissionswert ermittelten Remissionswerte beziehungsweise zu dem gleichen Remissionswert ermittelten Transmissionswerte zur Bestimmung des Mittelwertes verwendet. Auf diese Weise wird eine einfache Bestimmung des Referenzwertes ermöglicht und es fließen quasi automatisch alle Garnparameter in den Referenzwert ein. Durch die Mittelung über eine gewisse Länge des Garns, spielen Verunreinigungen, die während der Referenzlänge auftreten, für den Referenzwert praktisch keine Rolle.

Als Referenzlänge kann dabei nur ein erster Teil der Garnlänge oder die gesamte Garnlänge verwendet werden. Zur Kompensation der Garnparameter ist dabei in der Regel eine begrenzte Länge zur Referenzwertbestimmung ausreichend. Es spielen jedoch noch andere Einflussfaktoren, die die Messung beeinflussen, eine Rolle. Dazu zählen die Verschmutzung oder Alterung der verwendeten Lichtquelle oder der Sensoren für die Messung des transmittierten oder remittierten Lichtes oder die Veränderung des Farbspektrums der Lichtquelle sowie eine mögliche Änderung der Fadenposition. Da sich solche Einflüsse kontinuierlich ändern können, ist es von Vorteil, auch den Referenzwert kontinuierlich anzupassen. Das heißt die Mittelwertbildung wird in diesem Fall fortlaufend über die gesamte Garnlänge durchgeführt. Vorteilhafterweise wird der Mittelwert dabei gleitend gebildet.

Vorteilhafterweise ist die Detektion von Verunreinigungen während einer ersten Länge des Garns nicht aktiv, und zwar solange, bis ausreichend Remissionswerte für eine Mittelwertbildung zur Verfügung stehen. Je nachdem, wie die Referenzlänge festgelegt wird, werden damit während eines ersten Teils der Referenzlänge keine Verunreinigungen ausgewertet oder während der gesamten Referenzlänge.

Für die Berechnung des Mittelwertes kommen verschiedene Methoden in Frage. So kann zum Beispiel ein arithmetischer Mittelwert berechnet werden. Alternativ kann auch ein Median berechnet werden. Der Median hat dabei den Vorteil, dass Ausreißer besser kompensiert werden. Ein Exponentialfilter bietet ebenfalls die Möglichkeit der Mittelwertbildung und hat zudem den Vorteil, dass keine Anzahl von Remissions- oder Transmissionswerten zwischengespeichert werden muss.

Vorteilhafterweise werden der Transmissionswert beziehungsweise der Remissionswert und der zugeordnete Referenzwert in einer Tabelle abgelegt. Damit ist ein einfacher und schneller Zugriff auf die benötigten Werte sichergestellt. Eventuell in der Tabelle nicht abgelegte Werte können durch eine Funktion, insbesondere durch eine lineare Funktion, interpoliert werden.

Es kann entweder eine absolute oder eine relative Abweichung des aktuellen Remissionswertes beziehungsweise des aktuellen Transmissionswertes vom Referenzwert bestimmt werden. Eine Verunreinigung wird dann angezeigt, wenn die absolute oder relative Abweichung einen vorgegebenen Grenzwert über- oder unterschreitet. Wenn der aktuelle Remissionswert gegenüber dem remissionsabhängigen Mittelwert nach oben abweicht, liegt eine Hellstelle vor, weicht er nach unten ab, handelt es sich um eine Dunkelstelle.

In einer vorteilhaften Ausführungsform wird der aktuelle Remissionswert und der aktuelle Transmissionswert gleichzeitig ermittelt. Dadurch wird eine zeiteffiziente Detektion von Verunreinigungen beziehungsweise Fremdfasern ermöglicht und garantiert auch eine zuverlässige Fremdfasererkennung bei höheren Durchlaufgeschwindigkeiten des Garns, wie sie bei Spulmaschinen auftreten. Weiterhin ist auf einfache Weise sichergestellt, dass der aktuelle Remissions- und Transmissionswert auch an der gleichen Garnstelle gemessen werden.

Zur Lösung der Aufgabe wird ferner eine Vorrichtung zur Detektion von Verunreinigungen, insbesondere von Fremdfasern, in längsbewegtem Garn mit einer Steuer- und Auswerteeinrichtung vorgeschlagen, wobei die Steuer- und Auswerteeinrichtung dazu ausgebildet ist, dem jeweiligen Transmissionswert einen remissionsabhängigen Referenzwert zuzuordnen und den Referenzwert mit dem aktuellen Remissionswert zu vergleichen oder dem jeweiligen Remissionssignal einen transmissionsabhängigen Referenzwert zuzuordnen und den Referenzwert mit dem aktuellen Transmissionswert zu vergleichen

In einer vorteilhaften Ausgestaltung der erfindungsgemäßen Vorrichtung ist mittels der Steuer- und Auswerteeinrichtung der Referenzwert als Mittelwert von Remissionswerten beziehungsweise Transmissionswerten bestimmbar.

Entsprechend einer Weiterbildung der erfindungsgemäßen Vorrichtung ist die Steuer- und Auswerteeinrichtung dazu ausgebildet, den Mittelwert aus zu dem gleichen Transmissionswert ermittelten Remissionswerten beziehungsweise aus zu dem gleichen Remissionswert ermittelten Transmissionswerten zu bestimmen.

Vorteilhafterweise weist die Steuer- und Auswerteeinrichtung einen Speicher auf, in dem eine Tabelle, die jedem Transmissionswert beziehungsweise Remissionswert einen Referenzwert zuordnet, speicherbar ist.

Gemäß einer weiteren bevorzugten Ausführungsform sind der Sensor zur Erfassung des aktuellen Remissionswertes und der Sensor zur Erfassung des aktuellen Transmissionswertes so angeordnet sind, dass der Remissionswert und der aktuelle Transmissionswert gleichzeitig messbar sind.

Die Erfindung wird nachfolgend anhand von in den Zeichnungen dargestellten Ausführungsbeispielen näher erläutert.

Es zeigen:
- Fig. 1: eine erfindungsgemäße Vorrichtung;
- Fig. 2: eine grafische Darstellung der Remissionsmittelwerte in Abhängigkeit vom Transmissionswert für ein erstes Garn;
- Fig. 3: eine grafische Darstellung der Remissionsmittelwerte in Abhängigkeit vom Transmissionswert für ein zweites Garn;
- Fig. 4: eine Prinzipdarstellung der Bestimmung der absoluten Abweichung des Remissionswertes vom Remissionsmittelwert;
- Fig. 5: eine Prinzipdarstellung der Bestimmung der relativen Abweichung des Remissionswertes vom Remissionsmittelwert.
- Fig. 6: eine Prinzipdarstellung der Bestimmung der relativen Abweichung des Transmissionswertes vom Transmissionsmittelwert.

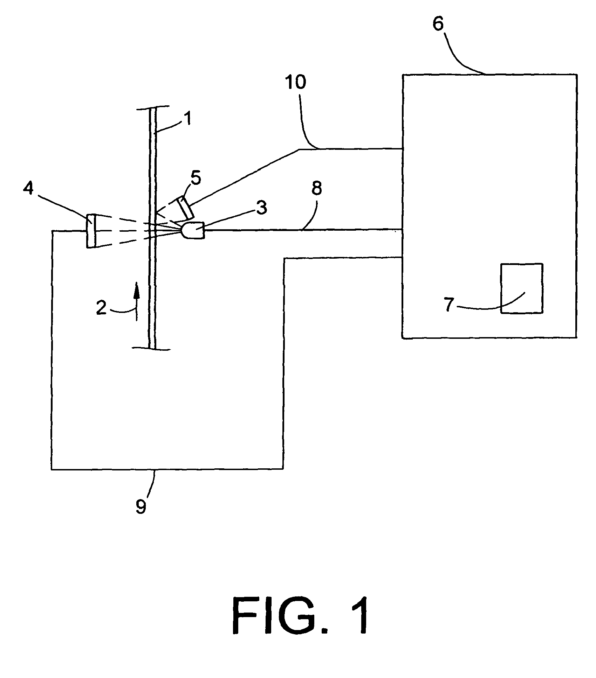

Fig. 1 zeigt eine mögliche Ausführungsform einer erfindungsgemäßen Vorrichtung. Das Garn 1 bewegt sich längs in Richtung des Pfeils 2. Die Lichtquelle 3, hier eine Leuchtdiode, emittiert Licht in Richtung des Garns 1. Das emittierte Licht wird vom Garn sowohl transmittiert als auch remittiert. Zur Messung der Intensitäten des transmittierten und des remittierten Lichtes weist die Vorrichtung zwei Sensoren 4 und 5 auf. Als Sensoren werden im dargestellten Ausführungsbeispiel Photodioden verwendet. Die Photodiode 4 misst dabei das vom Garn transmittierte Licht beziehungsweise die Abschattung. Die Photodiode 5 erfasst das vom Garn remittierte Licht. Die Vorrichtung beinhaltet ferner eine Steuer- und Auswerteeinrichtung 6 mit einem Speicher 7. In dem Speicher 7 ist eine Tabelle mit den Transmissionswerten und den zugehörigen Referenzwerten abgelegt. In dem darstellten Ausführungsbeispiel werden als Referenzwerte Remissionsmittelwerte verwandt. Die Steuer- und Auswerteeinrichtung ist zur Einstellung der Lichtstärke über die Leitung 8 mit der Leuchtdiode verbunden. Die Messsignale werden von den Photodioden 4 und 5 über die Leitungen 9 und 10 an die Steuer- und Auswerteeinrichtung 6 übertragen. Erfindungsgemäß wird von der Leuchtdiode 3 ein Lichtblitz ausgesandt und gleichzeitig mit der Photodiode 4 das Transmissionssignal und mit der Photodiode 5 das Remissionssignal gemessen. Die Intensität des Lichtblitzes bleibt dabei für alle Messungen konstant.

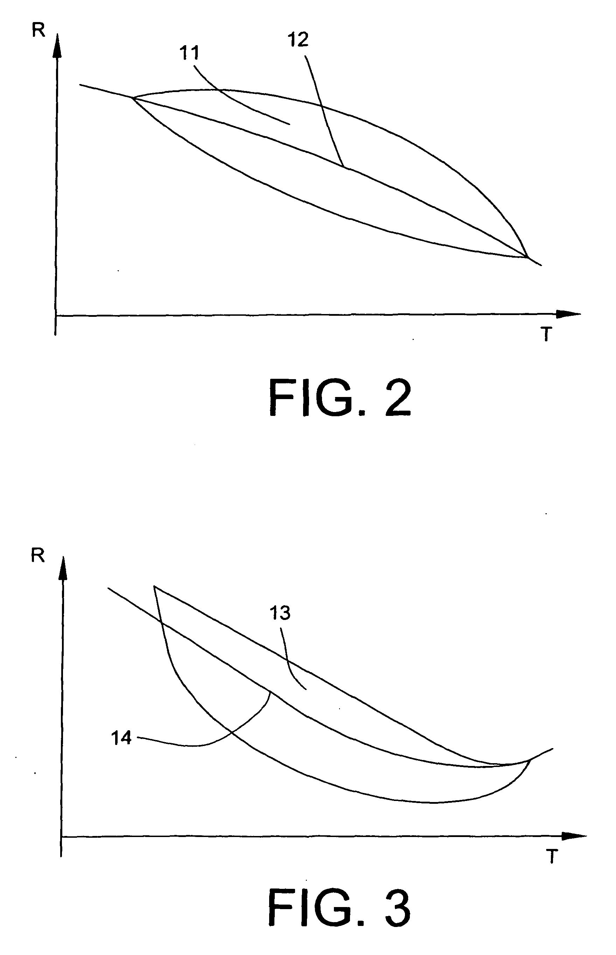

Die Figuren 2 und 3 zeigen eine grafische Darstellung der Remissionsmittelwerte in Abhängigkeit vom Transmissionswert für zwei verschiedene Garne mit unterschiedlichen Materialbeschaffenheiten. Zur Bestimmung der Mittelwerte werden eine Vielzahl von Remissionswerten in Abhängigkeit von den Transmissionswerten erfasst und die Mittelwerte werden kontinuierlich über die jeweils gewählte Referenzlänge des Garns mit jedem neuen Remissionswert angepasst. Aus der Vielzahl von Messungen ergibt sich für das erste Garn eine Punktewolke 11 und für das zweite Garn eine Punktewolke 13. Daraus ergeben sich die Remissionsmittelwerte in Abhängigkeit von den Transmissionswerten. Die Kurve 12 stellt die Mittelwerte für das erste Garn dar und die Kurve 14 veranschaulicht die Referenzwerte für das zweite Garn.

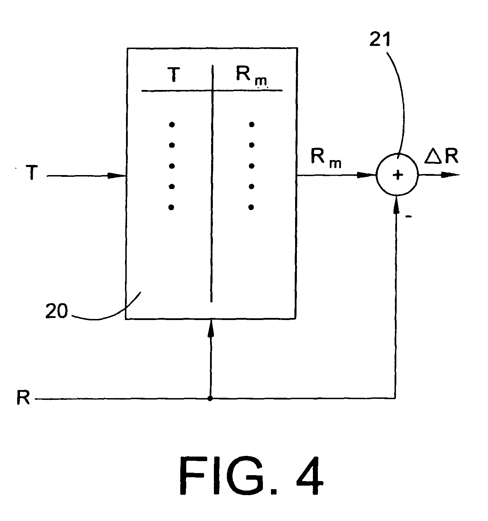

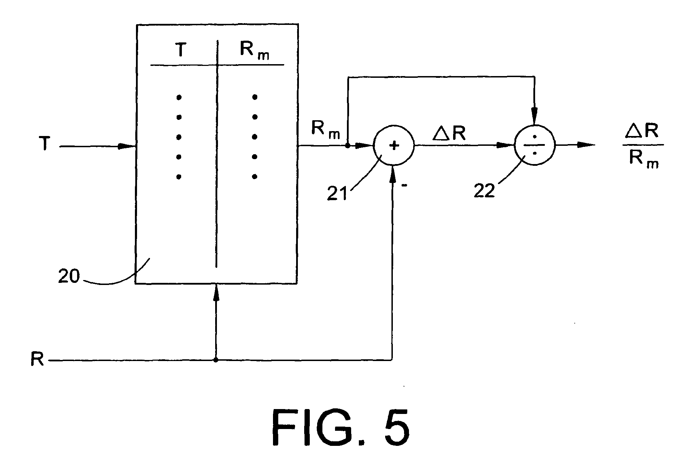

Die Figuren 4 und 5 zeigen eine Prinzipdarstellung der Bestimmung der absoluten und relativen Abweichung des Remissionswertes vom Remissionsmittelwert. Mit jedem Lichtblitz der Leuchtdiode 3 wird mittels der Photodioden 4 und 5 ein Transmissionswert T und ein Remissionswert R erfasst. Der Transmissionswert T wird der Tabelle 20 zugeführt, in der die Transmissionswerte und die zugehörigen Remissionsmittelwerte abgelegt sind. Als Ergebnis erhält man somit den Remissionsmittelwert Rₘ. Der neu gemessene aktuelle Remissionswert R_{λ} kann zur Verbesserung des Remissionsmittelwertes herangezogen werden. Zur Detektion von Verunreinigungen wird mittels des Addierers 21 der Remissionsmittelwert Rₘ und der mit (-1) beaufschlagte aktuelle Remissionswert R addiert. Dabei ergibt sich die Abweichung ΔR. Ist die Abweichung größer oder kleiner als ein vorbestimmter Wert wird eine Verunreinigung angezeigt. In Fig. 5 ist ergänzend die Berechnung einer relativen Abweichung ΔR/Rₘ vorgesehen. Dabei wird mittels des Dividierers 22 der Quotient aus der absoluten Abweichung ΔR und dem Remissionsmittelwertes Rₘ gebildet. Entsprechend kann diese relative Abweichung zur Detektion von Verunreinigungen herangezogen werden.

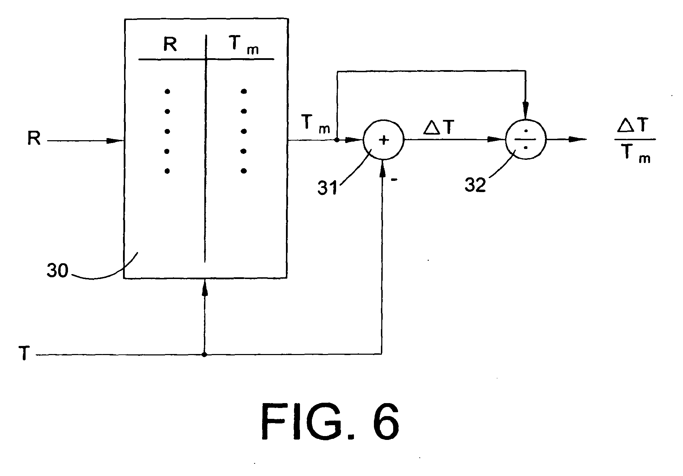

Fig. 6 zeigt eine weiteres Ausführungsbeispiel. Dabei wird nicht die Abweichung des aktuellen Remissionswertes vom Remissionsmittelwert bestimmt, sondern die Abweichung des aktuellen Transmissionswertes vom Transmissionsmittelwert. Analog zu den Figuren 2 und 3 wird zu jedem Remissionswert aus einer Vielzahl von Transmissionswerten ein Transmissionsmittelwert gebildet. Diese Werte werden in der Tabelle 30 abgelegt. Der aktuelle Messwert der Remission R wird der Tabelle 30 zugeführt. Daraus erhält man den zugehörigen Transmissionsmittelwert Tₘ. Neben dem aktuellen Remissionswert wird auch der aktuelle Transmissionswert T gemessen, der zur fortlaufenden Berechung des Transmissionsmittelwertes ebenfalls der Tabelle 30 zugeführt wird. Der Transmissionsmittelwert Tₘ und der mit (-1) beaufschlagte aktuelle Transmissionswert T werden dem Addierer 31 zugeführt und ergeben die Abweichung ΔT, die zur Bewertung von Verunreinigungen herangezogen werden kann. In Fig. 6 ist allerdings noch die Bestimmung des relativen Abweichung ΔT/Tₘ mittels des Dividierers 32 dargestellt, die alternativ zur Bewertung von Verunreinigungen herangezogen werden kann.

## Patentansprüche

1. Verfahren zur Detektion von Verunreinigungen, insbesondere von Fremdfasern, in längsbewegtem Garn (1), wobei Licht in Richtung des Garns emittiert wird, und wobei ein Remissionswert (R), der die Intensität des vom Garn remittierten Lichtes repräsentiert und ein zugehöriger Transmissionswert (T), der die Intensität des vom Garn transmittierten Lichtes repräsentiert, ermittelt wird, **dadurch gekennzeichnet, dass** dem jeweiligen Transmissionswert (T) ein remissionsabhängiger Referenzwert (Rₘ) zugeordnet wird und der aktuelle Remissionswert (R) mit dem Referenzwert (Rₘ) verglichen wird oder dass dem jeweiligen Remissionswert (R) ein transmissionsabhängiger Referenzwert (Tₘ) zugeordnet wird und der aktuelle Transmissionswert (T) mit dem Referenzwert (Tₘ) verglichen wird.

2. Verfahren nach Anspruch 1, **dadurch gekennzeichnet, dass** der remissionsabhängige Referenzwert ein Mittelwert (Rₘ) von Remissionswerten ist.

3. Verfahren nach Anspruch 1, **dadurch gekennzeichnet, dass** der transmissionsabhängige Referenzwert ein Mittelwert (Tₘ) von Transmissionswerten ist

4. Verfahren nach Anspruch 2 oder 3, **dadurch gekennzeichnet, dass** alle während einer Referenzlänge des Garns zu den gleichen Transmissionswerten (T) jeweils ermittelten Remissionswerte (R) oder zu den gleichen Remissionswerten (R) jeweils ermittelten Transmissionswerte (T) zur Bestimmung des Mittelwertes (Rₘ, Tₘ) verwendet werden.

5. Verfahren nach Anspruch 4, **dadurch gekennzeichnet, dass** als Referenzlänge ein erster Teil der Garnlänge verwendet wird.

6. Verfahren nach Anspruch 4, **dadurch gekennzeichnet, dass** als Referenzlänge die gesamte Garnlänge verwendet wird.

7. Verfahren nach einem der Ansprüche 4 bis 6, **dadurch gekennzeichnet, dass** während eines ersten Teils der Referenzlänge keine Verunreinigungen ausgewertet werden.

8. Verfahren nach Anspruch 5, **dadurch gekennzeichnet, dass** während der gesamten Referenzlänge keine Verunreinigungen ausgewertet werden.

9. Verfahren nach einem der Ansprüche 2 bis 8, **dadurch gekennzeichnet, dass** der Mittelwert (Rₘ,Tₘ) als arithmetischer Mittelwert berechnet wird.

10. Verfahren nach einem der Ansprüche 2 bis 8, **dadurch gekennzeichnet, dass** der Mittelwert (Rₘ,Tₘ) als Median berechnet wird.

11. Verfahren nach einem der Ansprüche 2 bis 8, **dadurch** gekenntzeichnet, dass der Mittelwert (Rₘ,Tₘ) durch ein Exponentialfilter gebildet wird.

12. Verfahren nach einem der Ansprüche 1 bis 11, **dadurch gekennzeichnet, dass** der Transmissionswert (T) und der zugeordnete Referenzwert (Rₘ) in einer Tabelle (20) abgelegt werden.

13. Verfahren nach einem der Ansprüche 1 bis 11, **dadurch gekennzeichnet, dass** der Remissionswert (R) und der zugeordnete Referenzwert (Tₘ) in einer Tabelle (30) abgelegt werden.

14. Verfahren nach einem der vorherigen Ansprüche, **dadurch gekennzeichnet, dass** eine absolute (ΔR, ΔT) oder relative (ΔR/Rₘ, ΔT/Tₘ) Abweichung des aktuellen Wertes (R,T) vom Referenzwert (Rₘ, Tₘ) bestimmt wird.

15. Verfahren nach Anspruch 14, **dadurch gekennzeichnet, dass** eine Verunreinigung angezeigt wird, wenn die absolute (ΔR, ΔT) oder relative (ΔR/Rₘ, ΔT/Tₘ) Abweichung einen vorgegebenen Grenzwert über- oder unterschreitet.

16. Verfahren nach einem der vorherigen Ansprüche, **dadurch gekennzeichnet, dass** der aktuelle Remissionswert (R) und der aktuelle Transmissionswert (T) gleichzeitig ermittelt werden.

17. Vorrichtung zur Durchführung des Verfahrens nach einem der Ansprüche 1 bis 16, mit einer Steuer- und Auswerteeinrichtung (6) und mindestens einer Lichtquelle (3), die dazu ausgebildet ist, Licht in Richtung des Garns zu emittieren, sowie Sensoren (4,5) zur Erfassung eines Remissionswertes (R), der die Intensität des vom Garn remittierten Lichtes repräsentiert, und eines zugehörigen Transmissionswertes (T), der die Intensität des vom Garn transmittierten Lichtes repräsentiert, **dadurch gekennzeichnet, dass** die Steuer- und Auswerteeinrichtung (6) dazu ausgebildet ist, dem jeweiligen Transmissionswert (T) einen remissionsabhängigen Referenzwert (Rₘ) zuzuordnen und den Referenzwert (Rₘ) mit dem aktuellen Remissionswert (R) zu vergleichen oder dass die Steuer- und Auswerteeinrichtung (6) dazu ausgebildet ist, dem jeweiligen Remissionswert (R) einen transmissionsabhängigen Referenzwert (Tₘ) zuzuordnen und den Referenzwert (Tₘ) mit dem aktuellen Transmissionswert (T) zu vergleichen.

18. Vorrichtung nach Anspruch 17, **dadurch gekennzeichnet, dass** mittels der Steuer- und Auswerteeinrichtung (6) der remissionsabhängige Referenzwert als Mittelwert (Rₘ) von Remissionswerten bestimmbar ist.

19. Vorrichtung nach Anspruch 17, **dadurch gekennzeichnet, dass** mittels der Steuer- und Auswerteeinrichtung (6) der transmissionsabhängige Referenzwert als Mittelwert (Tₘ) von Transmissionswerten bestimmbar ist

20. Vorrichtung nach Anspruch 18 oder 19, **dadurch gekennzeichnet, dass** die Steuer- und Auswerteeinrichtung (7) dazu ausgebildet ist, den Mittelwert (Rₘ, Tₘ) aus zu dem gleichen Transmissionswert (T) ermittelten Remissionswerten (R) oder aus zu den gleichen Remissionswert (R) ermittelten Transmissionswerten (T) zu bestimmen.

21. Vorrichtung nach einem der Ansprüche 17 bis 20, **dadurch gekennzeichnet, dass** die Steuer- und Auswerteeinrichtung (6) einen Speicher (7) aufweist, in dem eine Tabelle (20), die einem Transmissionswert (T) einen Referenzwert (Rₘ) zuordnet, speicherbar ist.

22. Vorrichtung nach einem der Ansprüche 17 bis 20, **dadurch gekennzeichnet, dass** die Steuer- und Auswerteeinrichtung (6) einen Speicher (7) aufweist, in dem eine Tabelle (30), die einem Remissionswert (R) einen Referenzwert (Tₘ) zuordnet, speicherbar ist.

23. Vorrichtung nach einem der Ansprüche 17 bis 22, **dadurch gekennzeichnet, dass** der Sensor (5) zur Erfassung des aktuellen Remissionswert (R) und der Sensor (4) zur Erfassung des aktuellen Transmissionswertes (T) so angeordnet sind, dass der aktuelle Remissionswert (R) und der aktuelle Transmissionswert (T) gleichzeitig messbar sind.

## Claims

1. Method for detecting impurities, in particular extraneous fibres, in longitudinally moved yarn (1), wherein light is emitted in the direction of the yarn, and wherein a reflectance value (R), which represents the intensity of the light reflected by the yarn and an associated transmittance value (T), which represents the intensity of the light transmitted by the yarn, are determined, **characterised in that** a reflectance-dependent reference value (Rₘ) is associated with the respective transmittance value (T) and the current reflectance value (R) is compared with the reference value (Rₘ) or **in that** a transmittance-dependent reference value (Tₘ) is associated with the respective reflectance value (R) and the current transmittance value (T) is compared with the reference value (Tₘ).

2. Method according to claim 1, **characterised in that** the reflectance-dependent reference value is a mean value (Rₘ) of reflectance values.

3. Method according to claim 1, **characterised in that** the transmittance-dependent reference value is a mean value (Tₘ) of transmittance values.

4. Method according to claim 2 or 3, **characterised in that** all the reflectance values (R) determined in each case during a reference length of the yarn with respect to the same transmittance values (T) or transmittance values (T) determined in each case with respect to the same reflectance values (R) are used to determine the mean value (Rₘ, Tₘ) .

5. Method according to claim 4, **characterised in that** a first part of the yarn length is used as the reference length.

6. Method according to claim 4, **characterised in that** the total yarn length is used as the reference length.

7. Method according to any one of claims 4 to 6, **characterised in that** no impurities are evaluated during a first part of the reference length.

8. Method according to claim 5, **characterised in that** no impurities are evaluated during the entire reference length.

9. Method according to any one of claims 2 to 8, **characterised in that** the mean value (Rₘ, Tₘ) is calculated as an arithmetic mean value.

10. Method according to any one of claims 2 to 8, **characterised in that** the mean value (Rₘ, Tₘ) is calculated as a median.

11. Method according to any one of claims 2 to 8, **characterised in that** the mean value (Rₘ, Tₘ) is formed by an exponential filter.

12. Method according to any one of claims 1 to 11, **characterised in that** the transmittance value (T) and the associated reference value (Rₘ) are entered in a table (20).

13. Method according to any one of claims 1 to 11, **characterised in that** the reflectance value (R) and the associated reference value (Tₘ) are entered in a table (30).

14. Method according to any one of the preceding claims, **characterised in that** an absolute (ΔR, ΔT) or relative (ΔR/Rₘ, ΔT/Tₘ) deviation of the current value (R, T) from the reference value (Rₘ, Tₘ) is determined.

15. Method according to claim 14, **characterised in that** an impurity is indicated when the absolute (ΔR, ΔT) or relative (ΔR/Rₘ, ΔT/Tₘ) deviation exceeds or falls below a predetermined limit value.

16. Method according to any one of the preceding claims, **characterised in that** the current reflectance value (R) and the current transmittance value (T) are determined simultaneously.

17. Device for carrying out the method according to any one of claims 1 to 16, with a control and evaluation mechanism (6) and at least one light source (3), which is configured to emit light in the direction of the yarn, as well as sensors (4, 5) for detecting a reflectance value (R), which represents the intensity of the light reflected by the yarn, and an associated transmittance value (T), which represents the intensity of the light transmitted by the yarn, **characterised in that** the control and evaluation mechanism (6) is configured to associate a reflectance-dependent reference value (Rₘ) with the respective transmittance value (T) and to compare the reference value (Rₘ) with the current reflectance value (R) or in that the control and evaluation mechanism (6) is configured to associate a transmittance-dependent reference value (Tₘ) with the respective reflectance value (R) and to compare the reference value (Tₘ) with the current transmittance value (T).

18. Device according to claim 17, **characterised in that** the reflectance-dependent reference value can be determined as the mean value (Rₘ) of reflectance values by means of the control and evaluation mechanism (6).

19. Device according to claim 17, **characterised in that** the transmittance-dependent reference value can be determined as the mean value (Tₘ) of transmittance values by means of the control and evaluation mechanism (6).

20. Device according to claim 18 or 19, **characterised in that** the control and evaluation mechanism (7) is configured to determine the mean value (Rₘ, Tₘ) from reflectance values (R) determined with respect to the same transmittance value (T) or from transmittance values (T) determined with respect to the same reflectance value (R).

21. Device according to any one of claims 17 to 20, **characterised in that** the control and evaluation mechanism (6) has a memory (7), in which a table (20), which associates a reference value (Rₘ) with a transmittance value (T), can be stored.

22. Device according to any one of claims 17 to 20, **characterised in that** the control and evaluation mechanism (6) has a memory (7), in which a table (30), which associates a reference value (Tₘ) with a reflectance value (R), can be stored.

23. Device according to any one of claims 17 to 22, **characterised in that** the sensor (5) for detecting the current reflectance value (R) and the sensor (4) for detecting the current transmittance value (T) are arranged in such a way that the current reflectance value (R) and the current transmittance value (T) can be measured simultaneously.

## Revendications

1. Procédé de détection d'impuretés, en particulier de fibres étrangères, dans un fil (1) déplacé longitudinalement, de la lumière étant émise en direction du fil et une valeur de réflectance (R) qui représente l'intensité de la lumière réfléchie par le fil et une valeur de transmission (T) associée qui représente l'intensité de la lumière transmise par le fil étant déterminées, **caractérisé en ce qu'**une valeur de référence (Rₘ) dépendante de la réflectance est associée à la valeur de transmission (T) respective et la valeur de réflectance (R) actuelle est comparée avec la valeur de référence (Rₘ) ou qu'une valeur de référence (Tₘ) dépendante de la transmission est associée à la valeur de réflectance (R) respective et la valeur de transmission (T) actuelle est comparée avec la valeur de référence (Tₘ).

2. Procédé selon la revendication 1, **caractérisé en ce que** la valeur de référence dépendante de la réflectance est une moyenne (Rₘ) de valeurs de réflectance.

3. Procédé selon la revendication 1, **caractérisé en ce que** la valeur de référence dépendante de la transmission est une moyenne (Tₘ) de valeurs de transmission.

4. Procédé selon la revendication 2 ou 3, **caractérisé en ce que** toutes les valeurs de réflectance (R) déterminées pour les mêmes valeurs de transmission (T) ou toutes les valeurs de transmission (T) déterminées pour les mêmes valeurs de réflectance (R) pendant une longueur de référence du fil sont utilisées pour déterminer la moyenne (Rₘ, Tₘ).

5. Procédé selon la revendication 4, **caractérisé en ce qu'**une première partie de la longueur du fil est utilisée comme longueur de référence.

6. Procédé selon la revendication 4, **caractérisé en ce que** toute la longueur du fil est utilisée comme longueur de référence.

7. Procédé selon une des revendications 4 à 6, **caractérisé en ce qu'**aucune impureté n'est évaluée pendant une première partie de la longueur de référence.

8. Procédé selon la revendication 5, **caractérisé en ce que** qu'aucune impureté n'est évaluée pendant toute la longueur de référence.

9. Procédé selon une des revendications 2 à 8, **caractérisé en ce que** la moyenne (Rₘ, Tₘ) est calculée sous forme de moyenne arithmétique.

10. Procédé selon une des revendications 2 à 8, **caractérisé en ce que** la moyenne (Rₘ, Tₘ) est calculée sous forme de médiane.

11. Procédé selon une des revendications 2 à 8, **caractérisé en ce que** la moyenne (Rₘ, Tₘ) est formée par un filtre exponentiel.

12. Procédé selon une des revendications 1 à 11 **caractérisé en ce que** la valeur de transmission (T) et la valeur de référence (Rₘ) associée sont enregistrées dans un tableau (20).

13. Procédé selon une des revendications 1 à 11, **caractérisé en ce que** la valeur de réflectance (R) et la valeur de référence (Tₘ) associée sont enregistrées dans un tableau (30).

14. Procédé selon une des revendications précédentes, **caractérisé en ce que** l'on détermine une différence absolue (ΔR, ΔT) ou relative (ΔR/Rₘ, ΔT/Tₘ) de la valeur actuelle (R, T) par rapport à la valeur de référence (Rₘ, Tₘ).

15. Procédé selon la revendication 14, **caractérisé en ce qu'**une impureté est indiquée quand la différence absolue (ΔR, ΔT) ou relative (ΔR/Rₘ, ΔT/Tₘ) devient supérieure ou inférieure à une valeur limite prédéfinie.

16. Procédé selon une des revendications précédentes, **caractérisé en ce que** l'on détermine en même temps la valeur de réflectance (R) actuelle et la valeur de transmission (T) actuelle.

17. Dispositif pour réaliser le procédé selon une des revendications 1 à 16, avec un dispositif de commande et d'évaluation (6) et au moins une source de lumière (3) qui est conçue pour émettre de la lumière en direction du fil, ainsi que des capteurs (4, 5) pour détecter une valeur de réflectance (R) qui représente l'intensité de la lumière réfléchie par le fil et une valeur de transmission (T) associée qui représente l'intensité de la lumière transmise par le fil, **caractérisé en ce que** le dispositif de commande et d'évaluation (6) est conçu pour associer une valeur de référence (Rₘ) dépendante de la réflectance à la valeur de transmission (T) respective et comparer la valeur de référence (Rₘ) avec la valeur de réflectance (R) actuelle ou que le dispositif de commande et d'évaluation (6) est conçu pour associer une valeur de référence (Tₘ) dépendante de la transmission à la valeur de réflectance (R) respective et comparer la valeur de référence (Tₘ) avec la valeur de transmission (T) actuelle.

18. Dispositif selon la revendication 17, **caractérisé en ce que** la valeur de référence dépendante de la réflectance peut être déterminée comme moyenne (Rₘ) de valeurs de réflectance au moyen du dispositif de commande et d'évaluation (6).

19. Dispositif selon la revendication 17, **caractérisé en ce que** la valeur de référence dépendante de la transmission peut être déterminée comme moyenne (Tₘ) de valeurs de transmission au moyen du dispositif de commande et d'évaluation (6).

20. Dispositif selon la revendication 18 ou 19, **caractérisé en ce que** le dispositif de commande et d'évaluation (7) est conçu pour déterminer la moyenne (Rₘ, Tₘ) à partir de valeurs de réflectance (R) déterminées pour la même valeur de transmission (T) ou à partir de valeurs de transmission (T) déterminées pour la valeur de réflectance (R).

21. Dispositif selon une des revendications 17 à 20, **caractérisé en ce que** le dispositif de commande et d'évaluation (6) présente une mémoire (7) dans laquelle peut être enregistré un tableau (20) qui associe une valeur de référence (Rₘ) à une valeur de transmission (T).

22. Dispositif selon une des revendications 17 à 20, **caractérisé en ce que** le dispositif de commande et d'évaluation (6) présente une mémoire (7) dans laquelle peut être enregistré un tableau (30) qui associe une valeur de référence (Tₘ) à une valeur de réflectance (R).

23. Dispositif selon une des revendications 17 à 22, **caractérisé en ce que** le capteur (5) pour détecter la valeur de réflectance (R) actuelle et le capteur (4) pour détecter la valeur de transmission (T) actuelle sont disposés de manière que la valeur de réflectance (R) actuelle et la valeur de transmission (T) actuelle soient mesurables en même temps.
